(19) Europäisches Patentamt
European Patent Office
Office européen des brevets

(11) Publication number: 0 065 354 B1

# (12) EUROPEAN PATENT SPECIFICATION

(45) Date of publication of patent specification: 03.07.85

(51) Int. Cl.⁴: C 07 C 33/46, C 07 C 29/62, C 07 C 29/66

(21) Application number: 82301964.1

(22) Date of filing: 16.04.82

## (54) Process for the preparation of 1,1-bis phenyl-2-haloalkan-1-ols.

(30) Priority: 12.05.81 GB 8114396
12.05.81 GB 8114391
20.08.81 GB 8125489
04.11.81 GB 8133317

(43) Date of publication of application:
24.11.82 Bulletin 82/47

(45) Publication of the grant of the patent:
03.07.85 Bulletin 85/27

(84) Designated Contracting States:
BE CH DE FR GB IT LI NL

(56) References cited:
DE-B-1 172 654
DE-C-1 076 112

(73) Proprietor: IMPERIAL CHEMICAL INDUSTRIES PLC
Imperial Chemical House Millbank
London SW1P 3JF (GB)

(72) Inventor: Falconer, James Alexander
15 Lawers Crescent Polmont
Falkirk Stirlingshire Scotland (GB)
Inventor: Fleming, Ian George Cameron
56 Newmains, Road
Kirkliston Scotland (GB)
Inventor: Jones, Raymond Vincent Heavon
26 Clarendon Road
Linlithgow Scotland (GB)
Inventor: Ewins, Robert Comgall
23 Tantallon Drive Carron
Falkirk Stirlingshire Scotland (GB)

(74) Representative: Houghton, Malcolm John et al
Imperial Chemical Industries PLC Legal
Department: Patents PO Box 6
Welwyn Garden City Herts, AL7 1HD (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 065 354 B1

**Description**

This invention relates to a process for the preparation of halohydrins which are 1,1-bis[(optionally substituted)phenyl]-2-haloalkan-1-ols.

DE—AS 1172654 describes and claims a process for the preparation of chlorohydrins by reacting an olefin or polyolefin with chlorine in an aqueous medium, characterised in that an olefin or polyolefin with 5 to 40 carbon atoms in the molecule, which can also be substituted, branched chain or cyclic, is reacted with chlorine in a liquid reaction medium which contains at least 50 parts by volume of an organic solvent boiling below 200°C and which is miscible with water as well as the olefin or polyolefin used, to 100 parts by volume of water.

The organic solvents used in the above process preferably have a boiling point below 150°C and particularly below 100°C, and the preferred solvents contain only carbon, hydrogen and oxygen in the molecule, for example, alcohols, ethers, aldehydes, ketones, carboxylic acids and carboxylic acid esters. The olefins employed as starting materials are typically hexadecene-1 and mixtures of $C_{13}$—$C_{15}$ alkenes.

EP—A—0015756 describes the preparation of certain fungicidal triazole compounds by reacting a compound of the general formula (A) or (B):

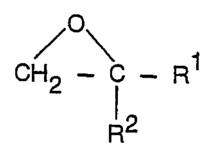

(A)

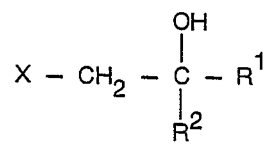

(B)

in which $R^1$ and $R^2$ have defined values, including phenyl with halogen substituents, and X is halogen, with 1,2,4-triazole. Mixtures of the compounds (A) and (B) are said to be obtained when a compound of the general formula (C) or (D):

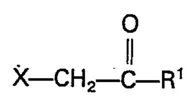

(C)

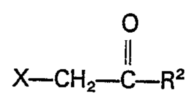

(D)

is reacted with a Grignard compound Y—Mg—$R^2$ or Y—Mg—$R^1$ in which Y is halogen. When $R^1$ in compound (C) is optionally substituted phenyl, compound (B) is said to predominate in the mixture.

According to the present invention there is provided a process for the preparation of halohydrins of the formula (I):

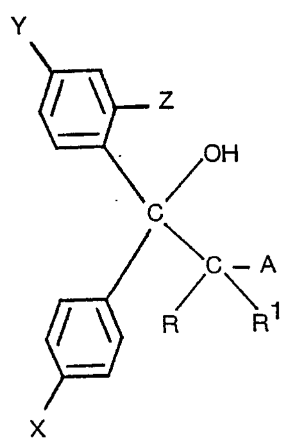

(I)

wherein R and $R^1$, which are the same or different, are hydrogen or alkyl, preferably $C_1$—$C_4$ alkyl; X, Y and Z are each independently hydrogen, fluorine or chlorine and A is chlorine or bromine, which comprises reacting a tertiary alcohol of the formula (II):

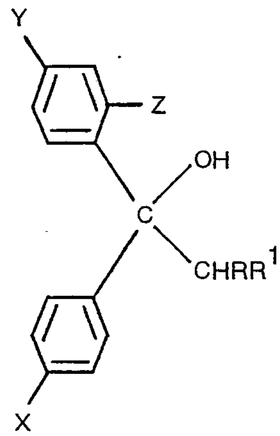

(II)

or a substituted alkene of the formula (III):

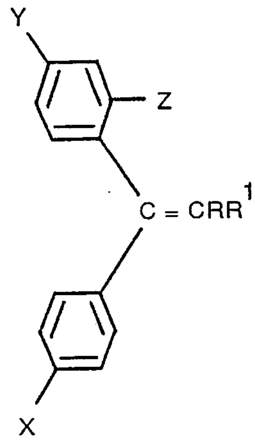

(III)

wherein R, $R^1$, X, Y and Z have the meanings defined above, with chlorine, bromine, hypochlorous acid or hypobromous acid in an aqueous medium.

When an alkene is used in the process, the aqueous medium is preferably a mixture of water and an amide solvent selected from diethylformamide, dimethylacetamide and preferably dimethylformamide.

The proportion of water to amide solvent is preferably such that the amount of water is the maximum which can be used without the reaction mixture forming two layers. This amount is, in general, approximately 25% w/w of the water and solvent taken together. If two layers are formed, the yields of halohydrin may be adversely affected.

In a particularly preferred process, the reaction is carried out in the presence of hydrogen peroxide. In this case the overall reaction can be represented by the following scheme:

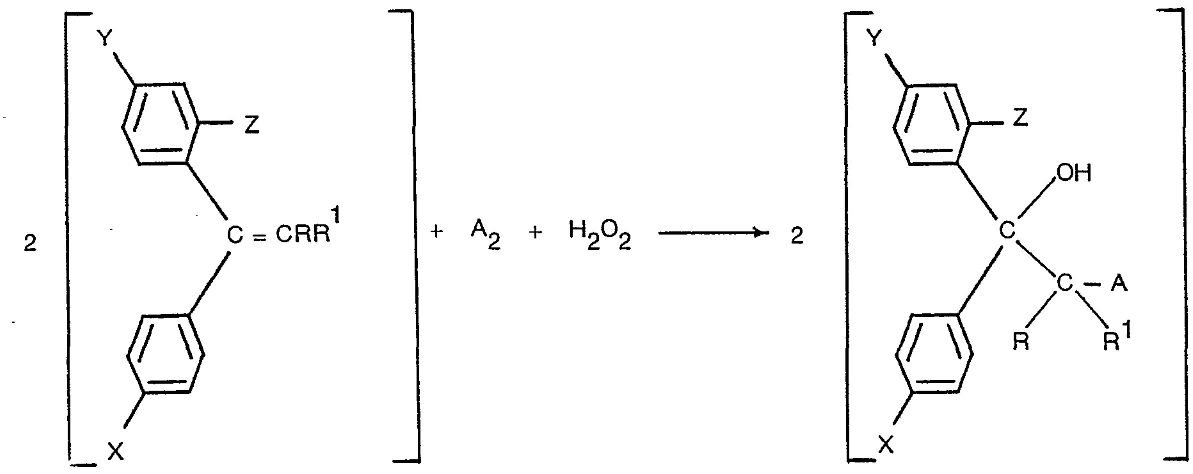

(III) (I)

from which it can be seen that 2 mols of alkene (II) require 1 mol of chlorine or bromine and 1 mol of hydrogen peroxide to give 2 mols of the halohydrin (I). This optimum theoretical stoichiometry may not be achievable in practice, but the particularly preferred process does ensure that a substantial part of the halogen employed is utilised in halohydrin formation.

The amount of chlorine or bromine which is used is preferably in slight excess over 1 mol per 2 mols of the alkene of formula (III), and is conveniently from 1.1 to 1.5 mols per 2 mols of the alkene.

Similar considerations apply to the amount of hydrogen peroxide, if used. In general, it is necessary to use more chlorine or bromine and hydrogen peroxide than is indicated by the above reaction scheme, probably due to the known reaction between chlorine or bromine and hydrogen peroxide removing some of these reactants from the system. The hydrogen peroxide is conveniently employed in the form of the commercially available 30% (100 volume) aqueous solution, although other strengths may be used if desired.

In the absence of an amide solvent, the process is carried out at a temperature of from 10 to 150°C and conveniently at the reflux temperature (ca. 100°C) of the aqueous reaction medium by slowly adding the chlorine, bromine, hypochlorous acid or hypobromous acid to a vigorously agitated mixture of water and the tertiary alcohol of formula (II) or the substituted alkene of formula (III), previously adjusted to a low pH by addition of an acid such as hydrochloric or sulphuric acid. A pH of about 1.0 is suitable and should not exceed 7. The product may then be isolated from the aqueous reaction mixture in known manner, for example, by extraction with a water-immiscible solvent and subsequent removal of the solvent from the extract by evaporation or distillation.

If hypochlorous acid or hypobromous acid are used in the process they may be added in the form of a dilute aqueous solution or they may, if desired, be generated *in situ* by addition of an aqueous solution of a salt such as an alkali metal salt, e.g. the sodium or potassium salt, or an alkaline earth metal salt, e.g. the calcium salt, of hypochlorous acid or hypobromous acid, to the reaction mixture. A particularly preferred salt of hypochlorous acid is sodium hypochlorite because of its commercial availability in the form of an aqueous solution.

Alternatively a mixture of a halide salt and an oxyhalide salt, for example, a mixture of a bromide and a bromate, may be used.

When an amide solvent is used, a reaction temperature from normal room temperature up to about 100°C is usual.

The reaction may be carried out by adding the alkene of formula (III) to a mixture of water and the amide solvent and then adding chlorine or bromine and, preferably, hydrogen peroxide to the mixture whilst maintaining the desired reaction temperature. A useful working concentration of alkene in the aqueous solvent mixture is about 15% w/w, although concentrations up to about 30% w/w and even higher may be used.

The reaction may conveniently be conducted by adding the alkene to the aqueous amide solvent and then adding the chlorine or bromine slowly, followed by the hydrogen peroxide, is used. Alternatively, the chlorine or bromine, and the hydrogen peroxide, may be added simultaneously or alternatively in portions to the alkene, amide solvent and water, or the alkene, chlorine or bromine and hydrogen peroxide may be added simultaneously or alternately in portions to the mixture of amide solvent and water. If, after all of the halogen has been added, a test of the reaction mixture shows that some alkene remains unreacted, further additions of chlorine or bromine and hydrogen peroxide may be made until all of the alkene has been used up. The product may separate out during the reaction, or if the proportions of amide solvent and water are such that this does not occur, the product may be isolated from the reaction mixture by dilution with water,

and usually forms an oil which may crystallise after a period of stirring at room temperature. The crude product is collected, washed with water and dried.

The process may be operated either batchwise or continuously.

The tertiary alcohols of formula (II) which may be used as starting materials in the present process may be obtained by a Grignard reaction between an alkyl magnesium halide of formula (IV):

$$RR^1HCMgHal \qquad (IV)$$

and a benzophenone of formula (V):

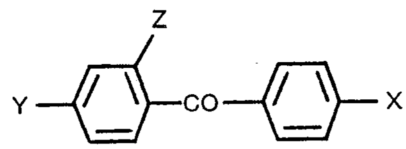

(V)

wherein R, $R^1$, X, Y and Z have the previously defined meanings and Hal is chlorine, bromine or iodine; under conditions known from the literature for the conduct of such reactions.

The benzophenones of formula (V) may be prepared in known manner by Friedel-Crafts reaction between a benzoyl halide of formula (VI):

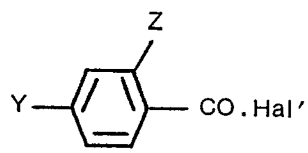

(VI)

and a benzene derivative of formula (VII):

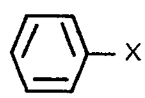

(VII)

wherein X, Y and Z have the previously defined meanings and Hal′ represents bromine or preferably chlorine, in the presence of a Lewis acid such as aluminium chloride.

Alternatively, the alcohols of formula (II) may be obtained by reaction in known manner of a compound of formula (VIII):

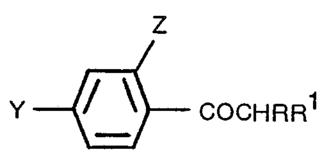

(VIII)

wherein R, $R^1$, Y and Z have the previously defined meanings with a Grignard compound of formula (IX):

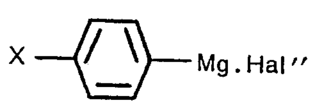

(IX)

wherein X has the previously defined meaning and HAl″ is bromine or iodine.

The compounds of formula (VIII) may be obtained by methods known from the art, for example, by Friedel-Crafts reaction of an acid halide of formula (X):

$$RR^1CH.COA \qquad (X)$$

wherein A, R and $R^1$ have the previously defined meanings, with a benzene derivative of formula (XI):

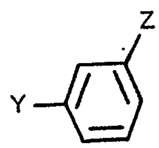

(XI)

in which Y and Z have the previously defined meanings.

Yet again, the tertiary alcohols of formula (II) in which X and Y are the same and Z is hydrogen may be prepared by reaction in known manner between 2 mol proportions of a Grignard compound of formula (IX) and 1 mol proportion of an ester of formula (XII):

$$RR^1.CH.COOR^2 \qquad (XII)$$

wherein R and $R^1$ have the previously defined meanings and $R^2$ is a hydrocarbon radical, preferably an alkyl radical and particularly a ($C_1$—$C_4$) alkyl radical. An example of an ester of formula (XII) which may be used is ethyl acetate.

The substituted alkenes of formula (III) which may also be used as starting materials in the present process may be obtained by dehydration of a tertiary alcohol of formula (II) using methods known from the literature for such dehydrations, for example, by heating the alcohol of formula (II) in the presence of a catalytic amount of a strong acid such as *p*-toluenesulphonic acid.

The substituted alkenes of formula (III) may also be obtained directly from the Grignard reaction between an alkyl magnesium halide of formula (IV) and a benzophenone of formula (V) as already indicated above, without isolation of the intermediate alcohol of formula (II). The Grignard reaction mixture, after completion of the reaction, is poured into dilute aqueous acid, and the organic layer collected. This organic layer contains the alcohol of formula (II) in the organic solvent or mixture of organic solvents, for example, a mixture of tetrahydrofuran and toluene, which was used in carrying out the preparation of the Grignard compound and its subsequent reaction with the benzophenone. A dehydrating agent is added to the organic layer and the whole heated to effect dehydration of the intermediate alcohol.

The halohydrins of formula (I) are useful intermediates for fungicides. Those in which R and $R^1$ are both hydrogen are intermediates for the preparation of fungicidal compounds of formula (XIII):

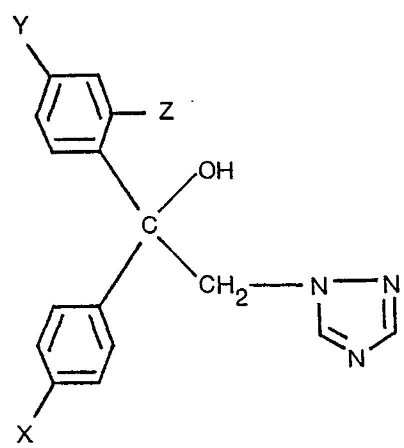

(XIII)

wherein X, Y and Z have the previously defined meanings.

Compounds of formula (XIII) and methods for their preparation including that from a halohydrin of formula (I), are disclosed in European Patent Application No. 0015756. Normally they will be obtained via the epoxide of formula (XIV):

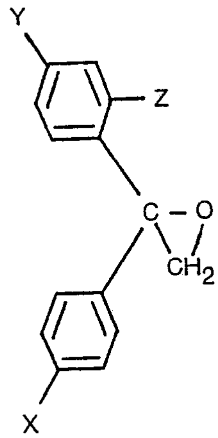

(XIV)

which is readily formed from the halohydrin under the normally basic conditions of the triazole addition.

Particularly useful halohydrins of formula (I) for the synthesis of fungicidal compounds of formula (XIII) are those in which R and $R^1$ are both hydrogen and at least one of X, Y and Z is chlorine or fluorine and especially in which R and $R^1$ are both hydrogen and X, Y and Z have the following substitution pattern:

| X | Y | Z |
|---|---|---|
| F | F | H |
| F | H | F |
| F | H | Cl |
| Cl | H | Cl |
| Cl | F | H |
| F | Cl | Cl |

The invention is illustrated by the following Examples in which parts and percentages are by weight.

Example 1

To a vigorously stirred mixture of 1,1-bis(4-fluorophenyl)ethanol (6 parts) in water (300 parts) at reflux (ca. 100°C) and pH 1.0 is added a diluted aqueous solution of commercial sodium hypochlorite (75 parts; ca 4% w/v available chlorine) over 2.5 hours. The pH after this addition is ca. 1.0. After cooling the product separates as an oil which is extracted with carbon tetrachloride. After removal of the solvent from the organic layer, the actual weight yield of 1,1-bis(4-fluorophenyl)-2-chloroethanol is 10.6 parts (strength 29.4%, determined by p.m.r. spectroscopy) which represents 47.4% of theory.

The 1,1-bis(4-fluorophenyl)ethanol used in this Example is prepared as follows:

A solution of methyl bromide (89 parts) in toluene (52 parts) is added, over about 30 minutes, to a stirred mixture of magnesium turnings (20 parts), toluene (156 parts) and tetrahydrofuran (122 parts), maintaining the temperature of 30—40°C. After the magnesium is dissolved 4,4'-difluorobenzophenone (168 parts) is added, whilst maintaining the temperature at 25—30°C. The mixture is drowned out into 5% aqueous ammonium chloride (1500 parts) and stirred for 30 minutes. The organic layer is separated, dried over anhydrous sodium sulphate and the solvent removed, under vacuum, at less than 40°C, to leave a brown oil (170 parts) containing 90% (by GLC, 10% SE30 at 200°C) of 1,1-bis(4-fluorophenyl)ethanol (85% conversion based on 4,4'-difluorobenzophenone). Distillation under vacuum, from sodium carbonate, gives 1,1-bis(4-fluorophenyl)ethanol contaminated with a small amount of 1,1-bis(4-fluorophenyl)ethene. Elution through silica gel, with toluene, removes the latter to leave a colourless liquid which is pure *1,1-bis(4-fluorophenyl)ethanol* (b.p. 103°/0.09 mm Hg; with slight decomposition).

*P.m.r. spectrum:* ($CDCl_3$/TMS): 1.90 (3H: singlet); 2.11 (1H: labile singlet); 6.80—7.60 (8H: aromatic complex).

4,4'-Difluorobenzophenone may be obtained, for example, as described in J.prakt.Chem., *135*, 245—266 (1932) or in European Patent Publication No. 4710.

## Example 2

To a mixture of 1,1-bis(4-fluorophenyl)ethene (28.5 parts) in water (1500 parts) (acidified to pH 1.0 with conc. hydrochloric acid) at reflux, is added sodium hypochlorite solution (400 parts) (2.6% w/v $Cl_2$) over 5 hours. The mixture is thoroughly extracted with carbon tetrachloride (200 parts) which is then washed with water (200 parts) and dried over anhydrous sodium sulphate. Removal of the solvent under vacuum leaves a brown, low melting point solid (36.5 parts) which contains 75.7% 1,1-bis(4-fluorophenyl)-2-chloroethanol (by quantitative PMR) (78% conversion based on 1,1-bis(4-fluorophenyl)ethene). Recrystallisation twice from petroleum ether (100—200°) and once from hexane gives fine white needles (19.9 parts) of pure *1,1-bis(4-fluorophenyl)-2-chloroethanol* (56.3% yield based on 1,1-bis(4,fluorophenyl)ethene (m.pt. 83—84°C).

| | | | | |
|---|---|---|---|---|
| (Found: | 62.3% C; | 4.3% H; | 13.4% Cl; | 14.3% F. |
| $C_{14}H_{11}ClF_2O$ requires: | 62.6% C; | 4.1% H; | 13.2% Cl; | 14.1% F). |

*P.m.r. spectrum*: ($CCl_4$/TMS): 2.99 (1H: labile singlet); 4.01 (2H: singlet); 6.80—7.50 (8H: aromatic complex).

The 1,1-bis(4-fluorophenyl)ethane used in this example may be prepared as described in J.Chem.Soc., 567—570 (1942).

## Example 3

To a mixture of 1,1-bis(4-fluorophenyl)ethene (28.5 parts) in water (1500 parts), at reflux, is added a solution of potassium bromide (36 parts) and bromine (28.8 parts) in water (295 parts), over 3 hours. The mixture is thoroughly extracted with carbon tetrachloride (200 parts) which is then washed with water (200 parts) and dried over anhydrous sodium sulphate. Removal of solvent under vacuum leaves a brown, low melting point solid (41.3 parts) which contains 69.3% (by PMR) 1,1-bis(4-fluorophenyl)-2-bromoethanol (70.4% conversion based on 1,1-bis-(4-fluorophenyl)ethene). Recrystallisation from petroleum ether (100—200°) gives fine white needles (20.4 parts) of pure *1,1-bis(4-fluorophenyl)-2-bromoethanol* (49.3% yield based on 1,1-bis(4-fluorophenyl)ethene (m.pt. 72.5—73.5°C).

| | | | | |
|---|---|---|---|---|
| (Found: | 53.6% C; | 3.5% H; | 12.1% F; | 25.2% Br. |
| $C_{14}H_{11}BrF_2O$ requires: | 53.7% C; | 3.5% H; | 12.1% F; | 25.5% Br). |

*P.m.r. spectrum:* ($CDCl_3$/TMS): 3.08 (1H: labile singlet); 4.03 (2H: singlet); 6.85—7.50 (8H: aromatic complex).

## Example 4

1-(2-Fluorophenyl)-1-(4-fluorophenyl)-2-bromoethanol

1-(2-Fluorophenyl)-1-(4-fluorophenyl) ethene (64.8 parts) was added slowly to a solution of sulphuric acid (27.5 parts) in water (500 parts) at 80°C; at the same time was added 114 parts by volume of a solution containing sodium bromide (22 parts) and sodium bromate (16 parts). Addition was complete 1 hour 40 minutes. After stirring at 80°C for a further 30 minutes, the mixture was stirred overnight, allowing the temperature to fall back to 20—25°C. A P.M.R. spectrum of the organic layer showed no residual alkene. The mixture was extracted once with chloroform (75 parts) and the lower layer separated off. Removal of the chloroform under vacuum gave a brown liquid (103.6 parts) containing 57.3 parts of 1-(2-fluorophenyl)-1-(4-fluorophenyl)-2-bromoethanol (61% of theoretical yield; analysis by H.P.L.C.).

## Example 5

A solution of 1,1-bis(4-fluorophenyl)ethene (23.5 parts), dimethylformamide (161 parts) and water (46 parts) is stirred at 44°C, and bromine (25 parts) is added over about 3 hours. Drowning into water (1500 parts) gives the product as an oil which crystallises on stirring at room temperature. Filtration, washing with water and drying at 60°C in vacuo gives crude product (33.6 parts) containing 96% (by quantitative PMR) 1,1-bis-(4-fluorophenyl)-2-bromoethanol [95% conversion based on 1,1-bis-(4-fluorophenyl)ethene]. A pure sample may be obtained by recrystallisation from petroleum ether (100—120°) and has m.p. 72.5—73.5°C.

## Example 6

A solution of 1,1-bis(4-fluorophenyl)ethene (46.7 parts), dimethyl formamide (388 parts) and water (113 parts) is stirred at 45°C, and chlorine (18.4 parts) is added over 5 hours. The solution is then cooled to 20°C and water (1000 parts) is added. An oil is formed which crystallises on stirring. The chlorohydrin is isolated by filtration and washed with water. After drying at 60°C in a vacuum oven, the crude chlorohydrin (60.3 parts) is obtained. This contains 76.5% (by PMR and GLC) 1,1-bis(4-chlorophenyl)-2-chloroethanol, which represents an 80% conversion from 1,1-bis(4-fluorophenyl)ethene. A pure sample may be obtained by recrystallisation from hexane and has m.p. 83—84°C.

The 1,1-bis(4-fluorophenyl)ethene used in these Examples may be prepared as described in J.Chem.Soc., 567—570 (1942).

Example 7

A solution of 1-(2-chlorophenyl)-1-(4-fluorophenyl)ethene (22 parts), dimethyl formamide (161 parts) and water (50 parts) is stirred at 60°C, and bromine (18.7 parts) is added over 45 minutes. After pouring the reaction mixture into water (500 parts), the product is extracted with chloroform (200 parts). After washing the extract with 10% hydrochloric acid (900 parts) and then with water (500 parts), the chloroform is removed under vacuo to leave crude product (32.0 parts) containing 72.7% (by quantitative pmr) 1-(2-chlorophenyl)-1-(4-fluorophenyl)-2-bromoethanol [74.6% conversion based on 1-(2-chlorophenyl)-1-(4-fluorophenyl)ethene].

Example 8

A solution of 1-(2-fluorophenyl)-1-(4-fluorophenyl)ethene (95 parts), dimethyl formamide (646 parts) and water (200 parts) is stirred at 60°C and bromine (80 parts) is added over 3 hours. After pouring the reaction mixture into water (2000 parts) and extracting with chloroform (500 parts), the extract is washed with 10% hydrochloric acid (2000 parts) and then with water (1000 parts). The chloroform is then removed under vacuo to leave the crude product (126.3 parts) containing 87.1% (by quantitative pmr) 1-(2-fluorophenyl)-1-(4-fluorophenyl)-2-bromoethanol, which represents an 87.5% conversion from 1-(2-fluorophenyl)-1-(4-fluorophenyl)ethene.

Example 9

A solution of 1,1-bis(4-fluorophenyl)ethene (43.2 parts) in dimethylformamide (280 parts) and water (80 parts) was stirred at 40°C, and bromine (18.8 parts) was added over 25 minutes, followed by 30% hydrogen peroxide solution (11.5 parts). A test showed 23% residual alkene. Further peroxide solution (24 parts) reduced the level of residual alkene to 14% and the reaction was completed by addition of bromine (4.7 parts). The mixture was cooled to 20°C and poured slowly into cold water (800 ml) to give white crystals. Filtration and air drying yielded crude product (61.2 parts) containing 94% (by HPLC) of 1,1-bis(4-fluorophenyl)-2-bromoethanol (91.9% conversion based on 1,1-bis(4-fluorophenyl)ethene).

Example 10

1-(2-Fluorophenyl)-1-(4-fluorophenyl)-2-bromoethanol

1-(2-Fluorophenyl)-1-(4-fluorophenyl)ethene (64.8 parts) was suspended in a mixture of N,N-dimethylformamide (72 parts) and water (40 parts), and the mixture heated to 30°C. Bromine (28.3 parts) and 30% hydrogen peroxide solution (18 parts) were added simultaneously over one hour. The mixture was stirred for a further 2 hours, when a P.M.R. spectrum of a melt sample showed no residual alkene. The mixture was heated to 60°C, held for 1 hour, and the lower layer run off. This yielded 129.1 parts of a pale brown liquid shown by chromatography (HPLC) to contain 76.8 parts of 1-(2-fluorophenyl)-1-(4-fluorophenyl)-2-bromoethanol. A sample of the 1-(2-fluorophenyl)-1-(4-fluorophenyl)-2-bromoethanol purified by HPLC was an oily liquid which decomposed on heating, and which did not give a distinct melting point. Combustion analysis gave C 53.5%; H 3.6%; F 12.8%; Br 26.3%; $C_{14}H_{11}BrF_2O$ requires C 53.67; H 3.54%; F 12.14%; Br 25.53%. P.M.R. spectrum ($CDCl_3$/TMS) 3.2 (1H, doublet, J = 1.8 Hz, $D_2$ exchangeable, labile, OH peak). 4.11 (1H, doublet J = 10.8 Hz, split into two doublets J = 1.8 Hz, olefinic H) 4.32 (1H, doublet J = 10.8 Hz, olefinic H); 6.85—7.8 (8H, aromatic complex).

Example 11

1-(2-Chlorophenyl)-1-(4-fluorophenyl)-2-bromoethanol

1-(2-Chlorophenyl)-1-(4-fluorophenyl)ethene (91.6 parts) was suspended in a mixture of N,N-dimethyl formamide (485 parts) and water (270 parts), and the mixture was heated to 40°C. Bromine (48.4 parts) and hydrogen peroxide 30% (30.6 parts) were added over approximately one hour in alternate aliquots (3.1 parts bromine, then 2 parts hydrogen peroxide). When addition was complete, the mixture was stirred overnight, allowing the temperature to fall back to 20—25°C. A test (P.M.R.) showed no residual alkene. Excess bromine was removed by addition of sodium bisulphite (5 parts). Agitation was stopped, and the lower layer was separated. This yielded 136.5 parts of a pale brown liquid, shown by P.M.R. analysis to contain 84.9 parts of 1-(2-chlorophenyl)-1-(4-fluorophenyl)-2-bromoethanol (64.5% of theoretical yield). P.M.R. ($CCL_4$/TMS) 4.15 (1H, doublet, J = 10.8 Hz, olefinic H); 4.34 (1H, doublet, J = 10.8 Hz, olefinic H), 4.5 (1H, broad peak, $D_2O$ exchangeable, labile, OH), 6.7—8.0 (8H, aromatic complex).

**Claims**

1. A process for the preparation of halohydrins of the formula (I):

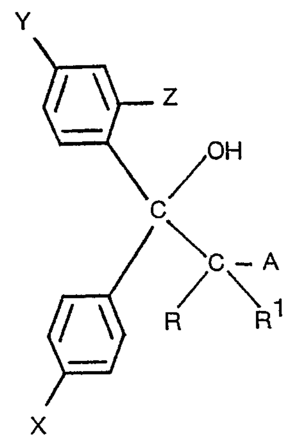

(I)

wherein R and $R^1$ which are the same or different, are hydrogen or alkyl; X, Y and Z are each independently hydrogen, fluorine or chlorine and A is chlorine or bromine, which comprises reacting a tertiary alcohol of the formula (II):

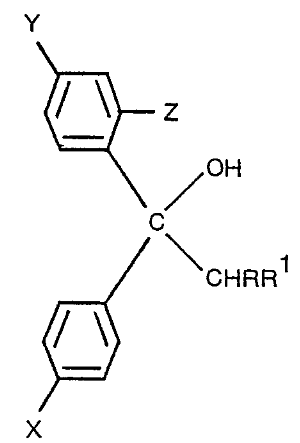

(II)

or a substituted alkene of the formula (III):

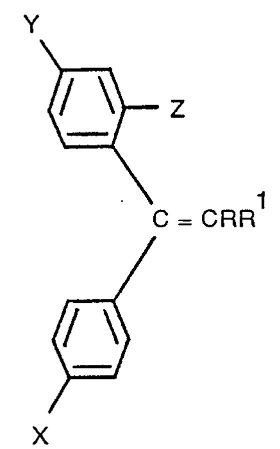

(III)

wherein R, $R^1$, X, Y and Z have the meanings defined above, with chlorine, bromine, hypochlorous acid or hypobromous acid in an aqueous medium.

2. A process for the preparation of halohydrins of the formula (I) in claim 1 which comprises reacting a substituted alkene of the formula (III) in claim 1 with chlorine or bromine in a mixture of water and an amide solvent selected from dimethyl formamide, diethyl formamide and dimethyl acetamide.

3. A process as claimed in claim 2 in which the reaction is carried out in the presence of hydrogen peroxide.

**Patentansprüche**

1. Verfahren zur Herstellung von Halogenhydrinen der Formel (I):

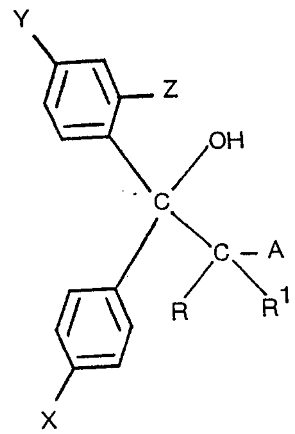

(I)

worin R und $R^1$, welche gleich oder verschieden sind, für Wasserstoff oder Alkyl stehen, X, Y und Z jeweils unabhängig für Wasserstoff, Fluor oder Chlor stehen und A für Chlor oder Brom steht, bei welchen ein tertiärer Alkohol der Formel (II):

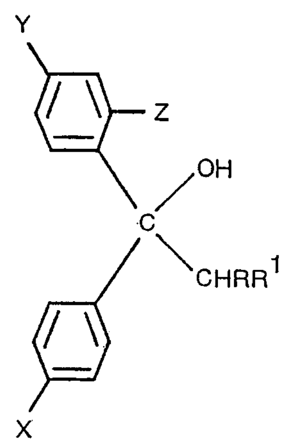

(II)

oder ein substituiertes Alken der Formel (III):

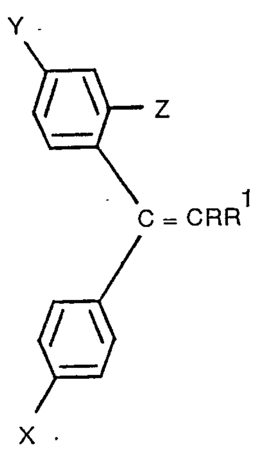

(III)

worin R, $R^1$, X, Y und Z die oben angegebenen Bedeutungen besitzen, mit Chlor, Brom, hypochloriger Säure oder hypobromiger Säure in einem wäßrigen Medium umgesetzt wird.

2. Verfahren zur Herstellung von Halogenhydrinen der Formel (I) von Anspruch 1, bei welchem ein substituiertes Alken der Formel (III) von Anspruch 1 mit Chlor oder Brom in einer Mischung aus Wasser und einem Amidlösungsmittel, das aus Dimethylformamid, Diäthylformamid und Dimethylacetamid ausgewählt ist, umgesetzt wird.

3. Verfahren nach Anspruch 2, bei welchem die Reaktion in Gegenwart von Wasserstoffperoxid ausgeführt wird.

## Revendications

1. Procédé de préparation d'halohydrines de formule (I):

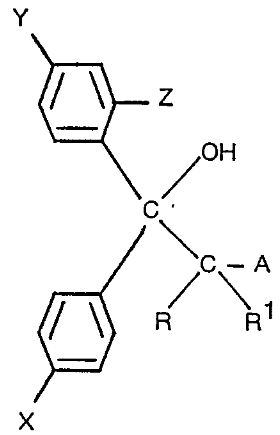

(I)

où R et $R^1$, qui sont identiques ou différents, représentent des atomes d'hydrogène ou radicaux alcoyle; X, Y et Z représentent chacun indépendamment des atomes d'hydrogène, de fluor ou de chlore et A représente un atome de chlore ou de brome, qui comprend la réaction d'un alcool tertiaire de formule (II):

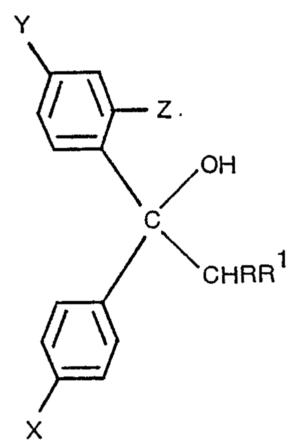

(II)

ou d'un alcène substitué de formule (III):

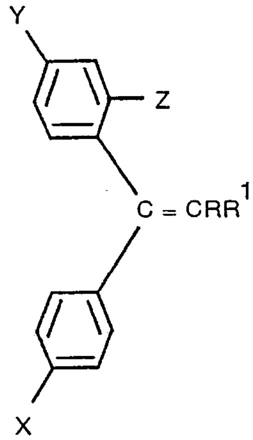

(III)

où R, $R^1$, X, Y et Z ont les significations définies ci-dessus, avec le chlore, le brome, l'acide hypochloreux ou l'acide hypobromeux dans un milieu aqueux.

2. Procédé de préparation d'halohydrines de formule (I) suivant la revendication 1, qui comprend la réaction d'un alcène substitué de formule (III) suivant la revendication 1 avec le chlore ou le brome dans un mélange d'eau et d'un solvant amidé choisi entre le diméthylformamide, le diéthylformamide et le diméthylacétamide.

3. Procédé suivant la revendication 2, dans lequel la réaction est exécutée en présence de peroxyde d'hydrogène.